Europäisches Patentamt

**European Patent Office**

Office européen des brevets

(11) Publication number: **0 005 013**
**B1**

## EUROPEAN PATENT SPECIFICATION

(45) Date of publication of patent specification: **12.05.82**

(21) Application number: **79300508.3**

(22) Date of filing: **29.03.79**

(51) Int. Cl.³: **A 61 K 6/04,** C 22 C 19/05, A 61 C 13/08

(54) **Dental restorative constructions and method of preparing same.**

(30) Priority: **30.03.78 US 891791**

(43) Date of publication of application:
**31.10.79 Bulletin 79/22**

(45) Publication of the grant of the patent:
**12.05.82 Bulletin 82/19**

(84) Designated Contracting States:
**BE CH DE FR GB IT LU NL SE**

(56) References cited:
**FR - A - 2 236 012**
**FR - A - 2 299 010**
**US - A - 3 914 867**
**US - A - 4 038 074**

**CHEMICAL ABSTRACTS, Vol. 87, No. 4,
published 25-07-1977. Columbus, Ohio, USA
SAJI KANZO et al.: "Nickel-chromium alloys
for dental crown casting", page 389, left-hand
column, abstract Nr. 29056e**

(73) Proprietor: **Johnson & Johnson**
**501 George Street**
**New Brunswick, New Jersey 08903 (US)**

(72) Inventor: **De Luca, Robert**
**108 Howard Way**
**Pennington New Jersey (US)**

(74) Representative: **Jones, Alan John et al,
CARPMAELS & RANSFORD 43 Bloomsbury
Square
London, WC1A 2RA (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Dental restorative constructions and method of preparing same

The invention relates to dental restorative constructions such as bridges, crowns and the like comprising a metal core of dental alloy and porcelain or other tooth simulating material bonded thereto, the dental alloys having good physical strength, corrosion resistance, workability and ability to bond to porcelain. The dental alloys have a base cost of substantially less than gold but are equal to or better than gold for many dental applications.

The metal cores of the dental construction are alloys composed primarily of nickel and chromium and containing on a weight percent basis, 65 to 80 per cent nickel, 12.0 to 20.0 per cent chromium, and, in addition, 3.5 to 5.0 per cent silicon, 3.0 to 6.0 per cent molybdenum, and 0.2 to 0.6 per cent boron. The alloys have excellent physical properties for dental applications, having a fusion temperature with the range of 1260°C to 1343°C (2300°F to 2450°F), a coefficient of expansion of from $13.6 \times 10^{-6} °C^{-1}$ to $14.5 \times 10^{-6} °C^{-1}$, good corrosion resistance when compared with similarly cast gold or other commercial non-precious metal dental alloys and good oxidation resistance. They also have, as cast, a tensile strength of at least 551,580 kPa (80,000 p.s.i.), an elongation of 2.0 to 5.0 per cent, a Rockwell hardness within the range of 90 to 100, and good bonding to porcelain.

The alloy has improved polishability as compared to the prior art non-precious metal alloys containing the same elements in different amounts. That is, the alloys polish almost as easily as gold alloys, yet the known advantages of non-precious metal alloys are retained.

Dental restorations such as bridges, crowns, dentures, partial dentures, inlays, onlays and the like have employed gold alloys for many years. Because of its high cost, many attempts have been made to make and employ non-precious metal alloys in place of the gold. Such non-precious metal alloy compositions are illustrated, for example by the U.S. Patents Nos. 1,736,053; 2,089,587; 2,156,757; 2,134,423; 2,162,252; 2,631,095; 3,121,629; 3,464,817; 3,544,315; and 3,914,867. Gold alloys, however, have many advantageous properties as a dental alloy and many of the previously prepared non-precious metal alloys have been found to be unsatisfactory in various respects when compared to the conventional gold alloy.

A dental alloy is disclosed in U.S. Patent No. 4,038,752 which is similar to the alloys used in the constructions of the invention. There is a difference in that the U.S. patent claims the use of copper, iron and manganese all of which could contribute to discoloration of the porcelain. Furthermore, at the upper limits of silicon claimed therein an alloy is provided which is too brittle and which would not have the improved polishability of the instant novel alloys. In fact, there is no teaching in this patent that would lead the skilled artisan to prepare an alloy having polishability which is similar to gold.

One of the problems encountered in attempts to use non-precious metal alloys for dental work in place of gold is that many of these alloys have been hard to cast because of a too high melting range. The alloy of the construction of the invention has, unlike some of the prior art alloys, a melting range which is suitable for use by the dental laboratory technician. For example, it will melt within the range of 2300 to 2450°F (1260°C to 1343°C). This melting range is desirable since many dental laboratories use torches of the gas oxygen type which will not heat too much above 2500°F (1370°C) so that if higher melting alloys are used then special heating equipment such as oxygen-acetylene torches must be obtained for working the metal. Although attempts have been made to adapt these alloys by modifying casting techniques such as by changing shape, dimensions, number and point of attachment of sprues, by using special investment materials, or by using special aftercasting treatments, the advantages of the heretofore available non-precious metal alloys have not been sufficient to serve toward general acceptance of these alloys as a preferred substitute for gold alloy in dental constructions.

Another problem with many of the heretofore known non-precious metal dental alloys is one of porcelain discoloration. In the firing process used to fuse the porcelain to the metal, certain coloring ions can diffuse into the porcelain, producing undesired colors in the procelain. Thus, for example, the presence of copper or iron in the metal alloy in an appreciable amount tends to discolor the porcelain bonded thereto. It is also expected that cobalt, present in a dental alloy, would be expected to discolor.

Also since non-precious metal dental alloy materials heretofore designed for use as structural metals are frequently substantially harder than gold, there is the added disadvantage of greater time and effort which must be spent on grinding the metal core for precise fit after casting.

In addition to the problems relative to the properties of the prior non-precious metal alloy as a general dental alloy, certain additional requirements exist in connection with the use of the dental alloy as a material which is to be faced with tooth enamel simulating material such as porcelain. Thus, the coefficient of expansion must be compatible with that of porcelain. Where there is not the desired compatibility in the coefficient of expansion between the metal and porcelain, fractures may develop in the porcelain during the firing and subsequent cooling. The preferred relationship of porcelain to metal is such that at room temperature there is compression in the porcelain or glass layer and tension in the metal. The alloys of the constructions of the invention when used as a core for dental prostheses, have been found to have higher thermal expansion coefficients and therefore will provide higher compression in the porcelain than dental alloys which are very similar in both the elements and the amounts which make up such alloys. Further, the fusion temperature of the alloy, while it should not be so high as to be difficult to cast, must be sufficiently above the firing temperature of the porcelain so that there is no deformation of the metallic core during firing. Moreover, metal alloys must bond adequately to porcelain so that when subjected to mechanical stress, there does not occur a separation at the interface in whole or in part. Thus, one object of this invention is to have an alloy with polishability comparable to gold and substantially better than the prior non-precious metal alloys.

It is also an object of the present invention to provide for dental constructions such as bridges and crowns, having a metal core of a non-precious metal alloy and a tooth enamel simulating outer covering bonded thereto wherein said non-precious metal alloy is free from the objections enumerated above and moreover the relationship of the physical properties between the metal core and outer covering are such that the foregoing problems are met. The preparation of a non-precious metal dental alloy which is particularly suitable in dental constructions is another object of the present invention. Another object is to provide for a dental construction which employs a non-precious metal alloy which is not only less costly than gold but has advantages over gold as a dental structural material. A further object is the provision of a dental alloy which may be employed in a dental construction without appreciably changing present techniques or equipment.

It has now been discovered that a dental construction comprising an appropriately contoured metal core of a non-precious metal alloy with a porcelain covering bonded thereto may be prepared which accomplishes the objects hereinbefore enumerated by employing for the metal core, a metal alloy having a fusion temperature within the range of from 1260°C to 1343°C (2300° to 2450°F), a coefficient of expansion of from $13.6 \times 10^{-6}°C^{-1}$ to $14.5 \times 10^{-6}°C^{-1}$ and a Rockwell B hardness as cast within the range of 90 to 100.

The expression "dental construction" as herein employed is meant a metal core of a non-precious metal alloy contoured in a desired form and at least one layer of porcelain bonded thereto. "Porcelain" as herein employed is meant dental porcelain as is known in the art and which is subsequently more fully described and illustrated. Normally in dental restorations porcelain is applied in several coatings and firings. In all coatings subsequent to the first coating, porcelain is bonded to porcelain. In the first coating, porcelain is bonded to metal and the problems to be solved are concerned particularly with the porcelain to metal relationship. Under present practice the porcelain which is bonded to metal is that understood in the art as opaque porcelain, as subsequently illustrated, but the present invention is not limited thereto. The expression "core" as herein employed is the metal framework or base, at least a portion of which is to be covered with porcelain. It may have any shape depending on the dental restoration intended; it is necessary only that a portion thereof is to have porcelain bonded thereto. The "coefficient of expansion" for the metal alloy as herein employed is the linear thermal expansion coefficient determined in the usual manner from values obtained on heating from room temperature up to 600°C at a rate of 7.5°C/minute.

The metal alloys in such core are prepared from nickel, chromium, silicon, molybdenum, and boron and contain on a weight percent basis, 65 to 80 per cent nickel, 12.0 to 20.0 per cent chromium, 3.5 to 5.0 per cent silicon, 3.0 to 6.0 per cent molybdenum, and 0.2 to 0.6 per cent boron. Preferably, the alloys contain 71 to 74.3 per cent nickel, 17.5 to 19.5 per cent chromium, 3.9 to 4.5 per cent silicon, 3.9 to 4.5 per cent molybdenum, and 0.3 to 0.5 per cent boron. The properties of the alloys, in addition to the fusion temperature, coefficient of expansion above recited, are good corrosion resistance, good oxidation resistance, a tensile strength of at least 551,580 kPa (80,000 p.s.i.), and a percent elongation of 2.0 to 5.0. The alloys bond well to porcelain and tensile strengths at the interface designated as porcelain bonding strength may be obtained which are greater than 34,474 kPa (5000 p.s.i.). Finally, the alloys show improved polishability over the non-precious alloys of the prior art. In fact, a lustre equivalent to gold is achieved with only slightly more effort than required to polish gold. The alloys polish with substantially less effort than required to polish prior art non-precious metal alloys such as disclosed in French Patent Specifications Nos. 2,236,012 and 2,299,010.

These properties are particularly advantageous for the preparation of the dental constructions and further in imparting desirable properties to the dental constructions made therefrom as hereinafter more fully described. The improved polishability is especially important to the dental technician since the time required to finish the dental prosthesis is directly proportional to how difficult it is to polish. The dental technician prefers something that polishes with as little effort as possible. Also the dental technician would like to have polishability equivalent to gold because it is a metal he is used to.

Detailed description of the invention

The dental construction of the present invention comprises an appropriately contoured core of a novel non-precious metal alloy with a porcelain covering bonded thereto. The metal alloys have a fusion temperature within the range of 1260°C to 1343°C (2300 to 2450°F) and a coefficient of expansion in the range of from $13.6 \times 10^{6-}°C^{-1}$ to $14.5 \times 10^{-6}°C^{-1}$ and consists of nickel, chromium and silicon, molybdenum and boron (i.e. apart from impurities).

The composition of the novel alloys is preferably from 71 to 74.3 per cent nickel, 17.5 to 19.5 per cent chromium, 3.9 to 4.5 per cent silicon, 3.9 to 4.5 per cent molybdenum and 0.3 to 0.5 per cent boron. Throughout the specification and claims, the percentages are on a weight basis.

O 005 013

These alloys have properties suitable for use as a structural metal in dental constructions, where they are to be faced with a tooth enamel simulating material and a dental construction of the alloy faced with a tooth enamel simulating material of porcelain constitutes the present invention. The alloys are especially useful for inlays, onlays and partial dentures. In addition to the above cited fusion temperatures and coefficient of expansion, other properties exhibited by the alloy include good corrosion resistance, good oxidation resistance, an ultimate tensile strength in the range of 551,580 kPa to 827,370 kPa (80,000 to 120,000 p.s.i.), a percent elongation of 2.0 to 5.0, and a Rockwell B hardness within the range of 90 to 100.

The fusion temperature range of 1260°C to 1343°C (2300 to 2450°F) of the alloys is within the range in which the dental laboratories are usually accustomed to work so that the alloy may be cast for the preparation of metal cores and other structural materials without change in equipment and technique. However, the fusion temperature is sufficiently high so that when the metal core is to be faced with porcelain it can resist deformation during the firing steps in porcelainization. Thus, the fusion properties of the alloys are ideally suited for dental construction to be faced with a tooth-simulating porcelain covering.

The coefficient of expansion in the range of $13.6 \times 10^{-6} °C^{-1}$ to $14.5 \times 10^{-6} °C^{-1}$ is suited to be employed with many dental porcelains. When suitable porcelains as subsequently more fully described are applied to the surfaces of the alloys, they are resistant to checking and other fractures which have tended to occur during the process of heating to the firing temperature followed by slow cooling to room temperature. Further, when employed with porcelain with expansion and contraction characteristics such that after cooling, the porcelain is under compression at room temperature, especially good results are obtained. Because the coefficient of expansion is slightly higher than the closely related prior art alloy of French Patent Specifications Nos. 2,236,012 and 2,299,010, it is expected that the porcelain would be in slightly more compression at room temperature. This is desirable in a dental prosthesis. Moreover, when bonded to porcelain, a good porcelain to metal bond is formed. The tensile strengths at the interface designated as the porcelain bonding strength may be obtained which are greater than 34,474 kPa (5000 p.s.i.).

In addition to the foregoing, another property important in its function as a metal core which is to be faced with porcelain is the absence of metals which form colored metal ions. Many metal alloys which may be desirable from strength and other properties frequently contain copper or iron and are therefore unsuitable for metal structure which is to be faced with porcelain. Cobalt is another metal which would be expected by those skilled in the art to cause discoloration when used in a dental alloy. The present alloys possess desirable properties without inclusion of such metals and a metal core of the present alloys may be faced with porcelain without staining.

In addition to the foregoing, other properties of the alloys render them superior as dental structural materials whether or not they are to be faced with porcelain. The alloys gave good strength, hardness and corrosion resistance while being light, stronger and harder than gold. It is recognized that the term good is relative, but as used herein it means good for the purposes of use in dentistry for which the material is specified. Thus, good strength and hardness as herein employed would be tensile strength and hardness above that of the gold alloys available at present. Good corrosion resistance would be resistance to etching by acid chloride solutions comparable to that shown by conventionally used dental alloys.

The ultimate tensile strength of 551,580 kPa to 827,370 kPa (80,000 to 120,000 p.s.i.) compares favourably with that of 448,159 kPa to 620,528 kPa (65,000 p.s.i. to 90,000 p.s.i.) for gold. The hardness of 90 to 100 compares favourably with the 86 Rockwell B hardness for gold. Thus, the alloys have strength and hardness superior to gold while having polishability similar thereto.

The good corrosion resistance of the alloys has been demonstrated by placing a sample of such alloy and a sample of the alloy disclosed in French Patent Specification No. 2,299,010 in a ferric chloride solution held at room temperature for three days. It was found that the corrosion resistance of the two alloys were similar.

In addition to the foregoing, the alloys can be remelted and cast without loss of their excellent physical properties, may be used with the dental solders presently employed when working with gold or with non-precious metal alloy solders, and may be faced with dental plastics materials such as the acrylics.

In the alloy compositions, not only is the actual amount of the metal component important but also the relationship of certain components to each other. One important relationship is that of chromium to nickel. When the chromium content with respect to the nickel is permitted to become too high, the thermal expansion of the alloy is found to be too low to obtain good matching with porcelain. Where the chromium content becomes too low, the alloy is generally found to have substantially poorer oxidation and corrosion resistance than desirable. A chromium to nickel ratio of 0.15 to 0.27 has been found to impart desirable properties to the alloy. A preferred ratio range is from 0.24 to 0.27. These ratios are to be considered together with the total nickel in the alloys.

As previously indicated, the silicon is preferably present in the range of 3.9 to 4.5 per cent by weight. When the silicon content is increased too much above 4.5 per cent, the alloy tends to become

4

brittle and lose some of its mechanical strength. When the silicon content is decreased too much below 3.9 per cent, the melting point and thermal expansion coefficient become too high.

The addition of molybdenum to the nickel, chromium and silicon stabilizes the thermal expansion property of the alloy against change which tends to occur during the repeated firing which are necessary procedures when porcelain is fused to the structural metal during the preparation of jackets, crowns and bridges. Further, molybdenum has the property of improving corrosion resistance.

Finally, molybdenum is necessary to improve the ductility of the alloys of the invention. This property is important because the polishability is related to ductility, and more importantly the dentist must be able to bend and otherwise make small adjustments to the material when he is placing a bridge in a patient's mouth.

Inclusion of boron improves the bonding strength between the alloy and porcelain. Hence, the alloy compositions contemplate the use of boron. However, it is critical and essential that boron shall not be employed in excess of 0.6 per cent by weight since boron tends to increase hardness, and thereby decrease the polishability of the alloys.

The alloy may be prepared in a conventional manner such as by placing the components in a fused alumina crucible and fusing the ingredients with appropriate mixing. While in the molten state, the alloy may be poured into moulds for ingot formation.

The dental alloys intended for use as structural metals may be employed in the replacement of the heavier and more expensive gold which has been the conventional structural metal used for dental purposes. The alloys are ideally suited for use where bonding of the alloy to porcelain is required, as in the preparation of artificial teeth, crowns, bridges and the like. The alloy may also be used in the preparation of porcelain veneers. Their physical properties also make the alloys highly useful for the preparation of metal crowns where the metal acts to completely cover the prepared tooth and for the preparation of inlays and onlays. In such usage the dental alloys are found to be not only as good as the gold which has heretofore been used but in many respects superior to such gold.

The dental constructions of the present invention may be obtained by first preparing an appropriately contoured metal core made of the alloy by casting according to conventional procedures and thereafter painting the metal core with porcelain and firing to secure the porcelain on the metal by bonding. Thereafter, additional layers of porcelain are applied with firing after each step to obtain an artificial tooth or other dental restoration.

By "porcelain" is meant dental porcelain as is known in the art and embraces dental glasses. They generally contain silicon oxide, aluminum oxide, sodium oxide, potassium oxides and minor amounts of other oxides. Normally, the porcelain covering which is first applied to the metal is an opaque porcelain. An opaque porcelain reduces the tendency of the metal to be seen through the final coating. Opaque porcelains are available commercially and include in the oxide composition either zirconium oxide, tin oxide, zinc oxide, titanium oxide, or zirconium silicate as an opacifying agent. The opaque porcelain is normally coated with additional layers of body porcelain followed by a layer of incisal porcelain under conventional procedures. The body porcelain is available commercially as gingival porcelain and may have a small amount of opacifying agent and incisal porcelain has no opacifying agent.

The exact composition of the porcelains is not critical although generally speaking, the porcelains are to be selected from those employing orthoclase feldspar as raw material. It is essential however, that certain properties be observed in the selection of an appropriate porcelain. Thus, the porcelains should have a fusion temperature maximum of 1010°C (1850°F) and a coefficient of thermal expansion in the range of $10^{-5}°C^{-1}$ to $21 \times 10^{-6}°C^{-1}$. It is recognized that a meaningful single coefficent of expansion is not obtainable for porcelain as it is for metal over the broad temperature range of from about room temperature up to 600°C and that coefficients of expansion values are valid only for a narrower range of temperatures. Porcelains which may be employed with the metal alloy of the present invention will be those whose several coefficients of expansion are within the above ranges when determined at several temperature ranges up to 575°—600°C.

Typical porcelain compositions are found in standard references such as Skinner and Phillips. "The Science of Dental Materials", p 518, W. B. Saunders Company, Philadelphia and London 1967; the compositions of several commercial porcelains are listed on page 60 of Jean-Marc Meyer, "Contributions a l'Etude de la Loaison Ceramometallique des Porcelaines cuites sur Alliages en Prosthese Dentaire", Thesis, University of Geneva, 1971. Suitable porcelains include those having compositions described in U.S. Patent No. 3,052,982 of the following oxide content: 61—67.8 per cent $SiO_2$; 11.7—17.1 per cent $Al_2O_3$; 0.1—2.6 per cent CaO; 0.1—1.8 per cent MgO; 2.37—9.6 per cent $Na_2O$ and 6.7—19.3 per cent $K_2O$. The foregoing composition may be modified to include lithium oxide in amounts up to 5 per cent and the other oxides reduced or modified. In addition, the porcelain may be modified to add from 0.05 to 25 per cent of an opacifying agent and the other oxides reduced or modified as desired limited by the need to keep the temperature and expansion properties within the desired limits. Suitable opaque porcelains may have the oxides in the following approximate ranges: $SiO_2$ 47 to 63 per cent; $Al_2O_3$ 10 to 14 per cent; CaO 0.6 to 1.3 per cent; $K_2O$ 8.5 to 11 per cent; $Na_2O$ 1.5 to 5 per cent; MgO 0.4 to 0.8 per cent; and $SnO_2$ 9 to 25 per cent. The present invention is not directed to the chemical composition of the porcelain, thus, any commercially available dental porcelain

5

or porcelain compositions prepared by a skilled artisan may be employed in the dental construction provided the foregoing properties are met.

From the porcelains of the type described above, particular porcelains may be selected for bonding to the alloys to obtain good bonding properties by empirical tests. One such test employs rods of alloy and porcelain of the same dimensions, preferably thin rods about 5.08 cm (2 inches) in length. The rods are heated from room temperature up to 600°C and the lengths measured at 575°C. Those porcelain rods whose lengths are within 6 per cent of the length of the alloy rods are considered to be a good match for purposes of providing a covering for the alloy with good bonding properties.

In carrying out the preparation of the dental construction of the present invention from the alloy, the metal core is formed by casting into casting investments which have previously been prepared by conventional procedures. Pellets or slugs of the metal alloy are placed in a crucible and heated in a conventional manner until the alloy melts. The alloy melt is cast employing conventional procedures and apparatus such as a centrifugal casting machine to obtain a casting contoured roughly to the shape desired for the core. The casting is recovered employing conventional procedures and then ground to the desired final shape, and dried.

After the grinding step, the areas of the metal core which is to receive porcelain is sandblasted with a quartz abrasive and the shoulders which are not to receive the porcelain are polished. The cores then are rinsed in distilled water, and dried. The core units are then ready to receive the porcelain.

Porcelain, preferably opaque porcelain such as of the composition previously described, is applied to the sand-blasted area of the metal core. The thus painted core or core units are (a) air dried, (b) placed into a furnace preheated to 649°C (1200°F) and (c) fired first under vacuum (26—29 inches of mercury or 880 to 982 mbar) by raising the temperature up to 927°C (1700°F) at a rate of 50 to 56°C/minute (90—100°F/minute) and then in air by breaking the vacuum and continuing the heating to 1004 to 1010°C (1840—1850°F) to obtain a dental construction comprising an appropriately contoured metal core having porcelain covering bonded thereto which is then removed from the furnace and cooled at ambient temperature.

Although good bonding is obtained between the metal alloy and porcelain having a coefficient of expansion hereinbefore specified, a superior chemical bond which imparts to the dental construction an even greater resistance to separation on stress may be provided by employing a bonding agent. The exact nature of the bonding agent is not critical. Any suitable bonding agent may be employed. One of the preferred bonding agents is an aluminium-boron bonding agent in an organic carrier. One composition is a 30 per cent composition of aluminium and boron in a 2:1 ratio in petrolatum.

When a bonding agent is employed, the procedural steps after the grinding of the casting is modified and may be carried out as described hereinafter. The ground core is cleaned ultrasonically with distilled water and dried. The bonding agent is then applied to that portion of the metal core which is to be coated with porcelain. The bonding agent is allowed to dry and fired on the core by placing the treated metal core in a furnace preheated to 649°C (1200°F) and thereafter raising the temperature of the furnace to 1010°C to (1850°F) in vacuum. The core is then removed from the furnace and allowed to cool to ambient temperature. After cooling, the excess bonding agent is mechanically removed, and the core thereafter cleaned and dried. Other suitable methods appropriate for the bonding agent chosen may also be employed.

Generally, additional layers of porcelains are fired onto the foregoing dental construction to obtain dental construction which is an aesthetically pleasing artifical tooth or other dental restoration. The additional layers of porcelains are provided by a gingival porcelain which forms the principal bulk of the body of the artificial tooth and an incisal porcelain which provides translucency to the outer tips. In carrying out the preparation of a dental construction which is an aesthetically pleasing dental restoration, several layers of gingival porcelain and thereafter layers of incisal porcelain are applied on the dental construction having a covering of opaque porcelain bonded thereto and fired by heating from 649°C to 943°C (1200°F to 1700°F) under vacuum (26"—29" Hg or 880—982 mbar) and further to 982°C (1800°F) in air, and then cooling at room temperature, in separate sequential operations. More than one firing may be necessary. The dental construction thus obtained is useful in dental prosthesis.

The following examples illustrate the invention.

Example 1

The following alloys were prepared and used for polishability tests.

|  | Table I | |
| --- | --- | --- |
|  | A | B |
| Nickel | 72.6 | 72.65 |
| Chromium | 19.0 | 18.6 |
| Molybdenum | 4.0 | 4.25 |
| Silicon | 4.0 | 3.90 |
| Boron | 0.4 | 0.35 |

These melts were prepared by air induction melting a 4.5 Kg charge consisting of Nickel pellets

# 0 005 013

(99.8 per cent carbonyl nickel), Chromium flake (99.1 per cent Cr), Molybdenum pellets (99.7 percent Mo), Molybdenum powder (99.8 per cent Mo), Silicon lumps (98.0 per cent Si) and ordinary nickel-boron alloy lumps (15—18 per cent B). This was heated to 1482°C (2700°F) until molten. The temperature was reduced to 1427°C (2600°F) and rods approximately 7.62 mm (0.300 inches) in diameter were aspiration cast with VYCOR* (Registered Trade Mark) glass or fused silica glass tubing.

Cut up rods were remelted with a gas-oxygen torch and cast in to investment moulds using dental laboratory techniques. They were easily melted and cast.

The surfaces of the castings made in the above step were ground with conventional dental laboratory stones and rubber wheels. They were polished with felt wheels and diamond polishing paste. Rouge was also used to polish these samples.

Both Alloy A and B were judged Easy to polish by dental laboratory technicians.

The polishability of these alloys should be compared to the alloys disclosed and polished in Example 4 of French Patent Specification No. 2,299,010 which were judged to be Moderate to Hard to polish in the same test.

The other properties of the above alloys are listed below compared with a white gold dental crown and bridge alloy.

Table II

| Alloy | A | B | White gold |
|---|---|---|---|
| Melting temp (°F) | 2360 | 2350 | 2300—2345 |
| Melting temp (°C) | 1293 | 1288 | 1260—1285 |
| Yield strength (psi) | 59,800 | 71,800 | 65,000 |
| Yield strength (MN/m²) | 412 | 495 | 448 |
| Ultimate strength (psi) | 96,200 | 96,750 | 88,300 |
| Ultimate strength (MN/m²) | 663 | 667 | 609 |
| Percentage elongation | 4.0 | 2.5 | 10 |
| Brinell Hardness | 190 | 200 | 200 |
| Castability | 67 microns* | 62 microns* | 73 microns* |
| Thermal Expansion Coefficient | $13.9\times10^{-6}°C^{-1}$ | $4.0\times10^{-6}°C^{-1}$ | $13.8\times10^{-6}°C^{-1}$ |

*1 micron$=1\times10^{-6}$ m.

From these data, it can be concluded that these alloys will perform satisfactorily in dental restoration.

Specific Examples of Deviation from Preferred Compositions

Other alloys similar to the above alloys were tested and it was found that less than 0.6 weight percent boron was necessary for a polishability property of Easy. Alloys with 0.8 percent boron were Moderate. Examination of the physical properties indicated that at least 0.2 weight percent boron is necessary to maintain high ultimate strength, low melting temperature and thermal expansion coefficient.

Similarly, it was found that the silicon must be kept above 3.0 percent to maintain the alloy yield strength above 413,686 kPa (60,000 psi). Alloys having more than 4.5 per cent silicon would have higher hardness values and be more difficult to polish.

Because molybdenum has a strong effect on ductility and is believed to impart corrosion resistance, its preferred range has been set at 3.9 to 4.5 per cent. Lower values tend to exhibit reduced elongation. Alloys high in molybdenum have higher melting temperature.

Example 2

Comparative polishability of alloy A, an alloy disclosed in French patent specification No. 2,299,010 and a Gold-base dental alloy

Experimental Procedure

Alloy A and the Alloy of French Patent Specification No. 2,299,010 (consisting essentially of 72 per cent Ni, 4 per cent Si, 19 per cent Cr and 1.3 per cent B by weight) were investment cast, ground and finished to $16^m/_m\times16^m/_m\times1.8^m/_m$, 240 grit surface finish. The gold-base dental alloy was BAK-ON White Gold (Registered Trade-Mark), available from Ceramco; Inc., 31—16 Hunters Point Avenue, Long Island City, New York, and was used in the following test as received. The dimensions were $16^m/_m\times16^m/_m\times1.0^m/_m$. One $16^m/_m\times16^m/_m$ face, of each alloy, was finished to 240 grit surface finish.

Hardness testing

The hardness of Alloy A and BAK-On White Gold was measured with a Clark Hardness Tester using a Rockwell B indenter at 100 kg load. The hardness of the other alloy was measured with the same hardness tester, using a Rockwell C indenter at 150 kg load. Rockwell B and C values were converted to Brinell Hardness using a conversion table.

Polishing

Alloy samples were attached to a bearing plate and polished with a Buehler Minimet Table Top Polisher. The polishing pad of the Minimet was impregnated with diamond polishing paste and the sample placed beneath the polishing arm. The polishing load was set to maximum and the polishing speed was set to 5. The polisher was started, allowed to run for the desired time and then stopped. The samples were polished for total times of 1, 2, 5, 10, and 15 minutes.

Reflectivity measurement

A Photovolt Reflection Density Unit 53A was used to make measurements of surface reflectivity. The diffuse reflectivity or density (D) was measured and the density data were converted to reflectivity (R) by the relation:

$$R = 1 - 100(10^{-D})$$

The density of the 240 grit surface was measured at five different locations. The readings were averaged and recorded. The sample was polished for one minute and remeasured. This procedure was repeated until fifteen minutes total polishing time were achieved.

The results, in the form of reflectivity versus polishing time are tabulated in Table 3. Table 3 shows that after five minutes polishing time, the reflectivity of Alloy A and BAK-ON White Gold reached 0.98 and increased only slightly with increased polishing time. Table 3 also shows that the Alloy of French Patent Specification No. 2,299,010 achieved a reflectivity of only 0.95 after five minutes polishing time and 0.98 after ten minutes. The Brinell Hardness of this Alloy was 326 compared with 255 and 182 for BAK-ON White Gold and Alloy A respectively. These results imply that there is a strong correlation between hardness and polishability.

### Table III
### Reflectivity versus polishing time for the alloy of French patent specification No. 2,299,010 BAK-ON White Gold and alloy A

| Polishing time (minutes) | The alloy of French patent specification No. 2,299,010 | Reflectivity BAK-ON white gold | Alloy A |
|---|---|---|---|
| 0 | .308 | .224 | .206 |
| 1 | .653 | .781 | .822 |
| 2 | .818 | .935 | .907 |
| 5 | .952 | .981 | .980 |
| 10 | .980 | .992 | .990 |
| 15 | .985 | .991 | .991 |

**Claims**

1. A dental restorative construction comprising a metal case of a non-precious metal alloy contoured in a desired form and a porcelain having a fusion temperature maximum of 1010°C and a coefficient of expansion in the range of from $10^{-5°}C^{-1}$ to $21 \times 10^{-6°}C^{-1}$ covering bonded thereto, said metal core being of an alloy having a fusion temperature within the range of 1260°C to 1343°C (2300°F to 2450°F), a coefficient of expansion in the range of from $13.6 \times 10^{-6°}C^{-1}$ to $14.5 \times 10^{-6°}C^{-1}$, a Rockwell B hardness as cast within the range of from 90 to 100, a tensile strength as cast of at least 551,580 kPa (80,000 psi), and an elongation as cast of 2.0 to 5.0 percent, and wherein said alloy consists of, on a weight basis, 65 to 80 percent nickel, 12 to 20 percent chromium, 3.5 to 5.0 percent silicon, 3.0 to 6.0 percent molybdenum, and 0.2 to 0.6 percent boron.

2. A dental restorative construction according to Claim 1 wherein said alloy consists of 71 to 74.3 percent nickel, 17.5 to 19.5 percent chromium, 3.9 to 4.5 percent silicon, 3.9 to 4.5 percent molybdenum and 0.3 to 0.5 percent boron.

3. A dental restorative construction according to Claim 1 or Claim 2 wherein the porcelain has a coefficient of expansion in the range of $10.33 \times 10^{-6°}C^{-1}$ to $20.25 \times 10^{-6°}C^{-1}$.

4. A method of preparing a dental restorative construction according to Claim 1 which comprises:
(a) preparing a metal core by casting a non-precious metal alloy having a fusion temperature within the range of 1260°C to 1343°C (2300 to 2450°F), a coefficient of expansion in the range of from $13.6 \times 10^{-6°}C^{-1}$ to $14.5 \times 10^{-6°}C^{-1}$, and which consists of, on a weight basis, 65 to 80 percent nickel, 12 to 20 percent chromium, 3.5 to 5.0 percent silicon, 3.0 to 6.0 molybdenum, and 0.2 to 0.6 percent boron, said alloy having a Rockwell B hardness as cast within the range of from 90 to 100, a tensile strength as cast of at least 551,580 kPa (80,000 p.s.i.), and an elongation as cast of 2.0 to 5.0 percent;

8

(b) applying to the surfaces of said metal core a porcelain having a fusion temperature maximum of 1010°C and a coefficient of expansion in the range of from $10^{-5}$°$C^{-1}$ to $21 \times 10^{-6}$°$C^{-1}$; and

(c) firing the porcelain onto said metal core.

5. A method according to Claim 4 wherein said alloy consists of 71 to 74.3 percent nickel, 17.5 to 19.5 percent chromium, 3.9 to 4.5 percent silicon, 3.9 to 4.5 percent molybdenum and 0.3 to 0.5 percent boron.

**Patentansprüche**

1. Wiederherstellende zahntechnische Anordnung, gekennzeichnet durch eine Metallkern aus einer Nichtedelmetallegierung in einer gewünschten Form sowie ein den Metallkern bedeckendes und daran gebundenes Porzellan mit einem maximalen Schmelzpunkt von 1010°C und einem Ausdehnungskoeffizienten von $10^{-5}$ bis $21 \times 10^{-6}$°$C^{-1}$, wobei die Legierung des Metallkerns einen Schmelzpunkt von 1260 bis 1343°C (2300 bis 2450°F), einen Ausdehnungskoeffizienten von $13,6 \times 10^{-6}$ bis $14,5 \times 10^{-6}$°$C^{-1}$, eine Rockwell-B-Härte nach dem Gießen von 90 bis 100, eine Zugfestigkeit nach dem Gießen von mindestens 551 580 kPa (80 000 psi) und eine Dehnung nach dem Gießen von 2,0 bis 5,0 % aufweist sowie aus 65 bis 80 Gew.-% Nickel, 12 bis 20 Gew.-% Chrom, 3,5 bis 5,0 Gew.-% Silicium, 3,0 bis 6,0 Gew.-% Molybdän und 0,2 bis 0,6 Gew.-% Bor besteht.

2. Wiederherstellende zahntechnische Anordnung nach Anspruch 1, dadurch gekennzeichnet, daß die Legierung aus 71 bis 74, 3 Gew.-% Nickel, 17,5 bis 19,5 Gew.-% Chrom, 3,9 bis 4,5 Gew.-% Silicium, 3,9 bis 4,5 Gew.-% Molybdän und 0,3 bis 0,5 Gew.-% Bor besteht.

Wiederherstellende zahntechnische Anordnung nach Anspurch 1 oder 2, dadurch gekennzeichnet, daß das Porzellan einen Ausdehnungskoeffizienten von $10,33 \times 10^{-6}$ bis $20,25 \times 10^{-6}$°$C^{-1}$ aufweist.

4. Verfahren zur Herstellung einer wiederherstellenden zahntechnischen Anordnung nach Anspruch 1, dadurch gekennzeichnet, daß man

(a) durch Gießen einer Nichtedelmetallegierung, die einen Schmelzpunkt von 1260 bis 1343°C (2300 bis 2450°F) und einen Ausdehnungskoeffizienten von $13,6 \times 10^{-6}$ bis $14,5 \times 10^{-6}$°$C^{-1}$ aufweist sowie aus 65 bis 80 Gew.-% Nickel, 12 bis 20 Gew.-% Chrom, 3,5 bis 5,0 Gew.-% Silicium, 3,0 bis 6,0 Gew.-% Molybdän und 0,2 bis 0,6 Gew.-% Bor besteht, einen Metallkern herstellt, wobei die Legierung nach dem Gießen eine Rockwell-B-Härte von 90 bis 100, eine Zugfestigkeit nach dem Gießen von mindestens 551 580 kPa (80 000 p.s.i.) and eine Dehnung nach dem Gießen von 2,0 bis 5,0 % hat,

(b) auf die Oberfläche des Metallkerns ein Porzellan mit einem maximalen Schmelzpunkt von 1010°C und einem Ausdehnungskoeffizienten von $10^{-5}$ bis $21 \times 10^{-6}$°$C^{-1}$ aufbringt sowie

(c) das Porzellan auf dem Metallkern brennt.

5. Verfahren nach Anspruch 4, dadurch gekennzeichnet, daß man eine Legierung aus 71 bis 74,3 Gew.-% Nickel, 17,5 bis 19,5 Gew.-% Chrom, 3,9 bis 4,5 Gew.-% Silicium, 3,9 bis 4,5 Gew.-% Molybdän und 0,3 bis 0,5 Gew.-% Bor einsetzt.

**Revendications**

1. Une construction pour restauration dentaire comprenant un noyau métallique en un alliage de métaux non préciux profilé sous une forme désirée et un revêtement de porcelaine ayant une température de fusion maximale de 1.010°C et un coefficient de dilation compris dans l'intervalle de $10^{-5}$°$C^{-1}$ à $21 \times 10^{-6}$°$C^{-1}$ qui y est collé, ledit noyau métallique étant en un alliage ayant une température de fusion comprise dans l'intervalle de 1.260°C à 1.343°C (2.300°F à 2.450°F), un coefficient de dilation compris entre $13,6 \times 10^{-6}$°$C^{-1}$ et $14,5 \times 10^{-6}$°$C^{-1}$, une dureté Rockwell B, à l'état brut de fonderie, comprise dans l'intervalle de 90 à 100, une résistance à la traction, à l'état brut de fonderie, d'au moins 551.580 kPa (80.000 livres par pouce carré) et un allongement, à l'état brut de fonderie, de 2,0 à 5,0 pour cent, et dans laquelle ledit alliage est composé, sur une base en poids, de 65 à 80 pour cent de nickel, de 12 à 20 pour cent de chrome, de 3,5 à 5,0 de silicium, de 3,0 à 6,0 pour cent de molybdène et de 0,2 à 0,6 pour cent de bore.

2. Une construction pour restauration dentaire selon la revendication 1 dans laquelle ledit alliage est composé de 71 à 74, 3 pour cent de nickel, de 17,5 à 19,5 pour cent de chrome, de 3,9 à 4,5 pour cent de silicium, de 3,9 à 4,5 pour cent de molybdène et de 0,3 à 0,5 pour cent de bore.

3. Une construction pour restauration dentaire selon la revendication 1 ou 2 dans laquelle la porcelaine a une coefficient de dilatation compris dans l'intervalle de $10,33 \times 10^{-6}$°$C^{-1}$ à $20,25 \times 10^{-6}$°$C^{-1}$.

4. Un procédé de préparation d'une construction pour restauration dentaire selon la revendication 1 qui consiste:

a) à préparer un noyau métallique en coulant un alliage de métaux non précieux ayant une température de fusion comprise dans l'intervalle de 1.260°C à 1.343°C (2.300 à 2.450°F), un coefficient de dilatation compris dans l'intervalle de $13,6 \times 10^{-6}$°$C^{-1}$ à $14,5 \times 10^{-6}$°$C^{-1}$ et qui est composé, sur une base en poids, de 65 à 80 pour cent de nickel, de 12 à 20 pour cent de chrome, de 3,5 à 5,0 pour cent de nickel, de 3,0 à 6,0 pour cent de molybdène et de 0,2 à 0,6 pour cent de bore, ledit alliage ayant une dureté Rockwell B, à l'état brut de fonderie, comprise dans l'intervalle de 90 à 100, une résistance à la traction, à l'état brut de fonderie, d'au moins 551.580 kPa (80.000 livres par pouce carré) et un allongement à l'état brut de fonderie de 2,0 à 5,0 pour cent;

b) à appliquer sur les surfaces dudit noyau métallique une porcelaine ayant une température de fusion maximale de 1.010°C et un coefficient de dilatation compris dans l'intervalle de $10^{-5}$°C$^{-1}$ à $21 \times 10^{-6}$°C$^{-1}$; et

c) à cuire la porcelaine sur ledit noyau métallique.

5. Un procédé selon la revendication 4 dans lequel ledit alliage est composé de 71 à 74,3 pour cent de nickel, de 17,5 à 19,5 pour cent de chrome, de 3,9 à 4,5 pour cent de silicium, de 3,9 à 4,5 pour cent de molybdène et de 0,3 à 0,5 pour cent de bore.